# EUROPEAN PATENT APPLICATION

(11) **EP 4 018 950 A1**
(43) Date of publication of application: **29.06.2022**
(21) Application number: 21216449.5
(22) Date of filing: 21.12.2021
(51) Int. Cl.: A61B 18/12, A61B 18/14, A61B 34/00, A61B 34/20, A61B 90/00, A61B 18/00

(54) **IMPROVING LESION UNIFORMITY IN BIPOLAR CARDIAC ABLATION**

(30) Priority: 22.12.2020 US 202017130007
(71) Applicant: Biosense Webster (Israel) Ltd, Yokneam, 2066717 (IL)
(72) Inventor: ALTMANN, Andres Claudio, 2066717 Yokneam (IL); GOVARI, Assaf, 2066717 Yokneam (IL); OZERI, Ella, 2066717 Yokneam (IL); MARZIANO, Lilah, 2066717 Yokneam (IL)
(74) Representative: Carpmaels & Ransford LLP

(57) **Abstract**

A method includes inserting into an organ a catheter having multiple electrodes, and placing the electrodes in contact with tissue for ablation. A selection from among the electrodes is received, including (i) first and second electrodes defining a first section of the catheter having a plurality of the electrodes, and (ii) third and fourth electrodes defining a second section of the catheter having at least two of the electrodes. Identifying whether the first and second sections jointly form a single contiguous ablation line or two disjoint ablation lines. A first set of ablation pulses is applied to the electrodes of the first and second sections, in case the first and second sections form the single contiguous ablation line, or a second different set of ablation pulses is applied to the electrodes of the first and second sections, in case the first and second sections form the two disjoint ablation lines.

## Description

### FIELD OF THE INVENTION

The present invention relates generally to medical devices, and particularly to methods and systems for improving lesion uniformity in bipolar cardiac ablation procedures.

### BACKGROUND OF THE INVENTION

Various techniques have been published for producing a uniform lesion in tissue ablation using a catheter having a plurality of electrodes.

U.S. Patent Application Publication No. 2013/0066316 describes a catheter and catheter system for eccentric remodeling and/or removal of atherosclerotic material of a blood vessel of a patient including an elongate flexible catheter body with a radially expandable structure. A plurality of electrodes or other electrosurgical energy delivery surfaces can radially engage atherosclerotic material when the structure expands.

U.S. Patent Application Publication No. 2001/0008967 describes an apparatus for delivering energy to a biological site, the apparatus includes an electrode device having a plurality of electrodes, the electrode device being positioned proximal the biological site. A power control system supplies power having a controllable phase angle to each of the electrodes. A backplate is also positioned proximal the biological site so that the biological site is interposed between the electrode device and the backplate. The backplate is maintained at the reference voltage level in relation to the power. The power control system controls the phase angle of the power so that the current flow between the electrodes and between the electrodes and the backplate results in the continuity and depth of lesions desired. In a preferred embodiment, the electrodes are arranged in a substantially linear array.

### SUMMARY OF THE INVENTION

An embodiment of the present invention that is described herein provides a method, including inserting into a patient organ a catheter having multiple electrodes, and placing at least some of the electrodes in contact with tissue of the organ for ablating the tissue. A selection is received from a user, the selection includes (i) first and second electrodes selected from among the electrodes, the first and second electrodes defining a first section of the catheter having two or more of the electrodes, and (ii) third and fourth electrodes selected from among the electrodes, the third and fourth electrodes defining a second section of the catheter having at least two of the electrodes. Identifying whether the first and second sections jointly form a single contiguous ablation line or two disjoint ablation lines. A first set of ablation pulses is applied to the electrodes of the first and second sections, in case the first and second sections form the single contiguous ablation line, or a second different set of ablation pulses is applied to the electrodes of the first and second sections, in case the first and second sections form the two disjoint ablation lines.

In some embodiments, applying the first set of ablation pulses includes delivering a first total energy via the second and third electrodes, and applying the second set of ablation pulses includes delivering via the second and third electrodes a second total energy, higher than the first total energy. In other embodiments, the first and second electrodes are positioned at edges of the first section, and the third and fourth electrodes are positioned at edges of the second section. In yet other embodiments, when the first and second sections form the single contiguous ablation line, one or more of the electrodes are common to the first and second sections.

In an embodiment, the second and third electrodes include the same electrode. In another embodiment, when the first and second sections form the single contiguous ablation line, the second electrode is adjacent to the third electrode. In yet another embodiment, the patient organ includes a heart.

There is additionally provided, in accordance with an embodiment of the present invention, a system, including an interface and a processor. The interface is configured to receive from a user a selection of (i) first and second electrodes selected from among electrodes of a catheter, such that at least some of the electrodes are in contact with tissue of an organ for ablating the tissue, the first and second electrodes defining a first section of the catheter having two or more of the electrodes, and (ii) third and fourth electrodes selected from among the electrodes, the third and fourth electrodes defining a second section of the catheter having at least two of the electrodes. The processor is configured to: (i) identify whether the first and second sections jointly form a single contiguous ablation line or two disjoint ablation lines, and (ii) control a pulse generator to apply to the electrodes of the first and second sections (a) a first set of ablation pulses, in case the first and second sections form the single contiguous ablation line, or (b) a second different set of ablation pulses, in case the first and second sections form the two disjoint ablation lines.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a catheter-based position-tracking and ablation system, in accordance with an exemplary embodiment of the present invention;
Figs. 2 and 3 are schematic, pictorial illustrations of sections of an ablation catheter, which comprise electrodes selected for applying bipolar ablations pulses to tissue, in accordance with exemplary embodiments of the present invention; and
Fig. 4 is a flow chart that schematically illustrates a method for performing a bipolar RF ablation procedure, in accordance with an exemplary embodiment of the present invention.

### DETAILED DESCRIPTION OF EMBODIMENTS

### OVERVIEW

In bipolar ablation procedures, a physician may select multiple electrodes of an ablation catheter, for defining multiple sections of the catheter that form one or more ablation lines. In some cases, selecting multiple sections of the catheter, may result in a formation of non-uniform ablation lines. For example, in case two of the sections have at least one common electrode, the common electrode may receive excess energy, which may result in a non-uniform ablation line.

Embodiments of the present invention that are described hereinbelow provide techniques for obtaining improved uniformity of the ablation line in bipolar ablation procedures, such as but not limited to radiofrequency (RF) ablation and irreversible electroporation (IRE) in patient heart.

In some embodiments, a system for bipolar ablation comprises a catheter having multiple ablation electrodes that, when placed in contact with tissue of the patient heart, are configured to apply ablation pulses to the tissue. The system comprises an interface, which is configured to receive from a user, e.g., a physician, a selection of electrodes (from the catheter electrodes) defining sections of the catheter. In the present example, the interface receives from the physician (i) first and second electrodes defining a first section of the catheter having two or more of the electrodes, and (ii) third and fourth electrodes defining a second section of the catheter, also having two or more of the electrodes.

In some embodiments, the system comprises a pulse generator, which is configured to apply ablation pulses to the tissue, in the present example, bipolar ablation pulses applied between two selected electrodes of the catheter.

In some embodiments, the system comprises a processor, which is configured to identify whether the first and second sections jointly form a single contiguous ablation line or two disjoint ablation lines. The processor is further configured to control the pulse generator to apply the bipolar ablation pulses to the electrodes of the first and second sections.

In some embodiments, in case the first and second sections form the single contiguous ablation line, the processor is configured to control the pulse generator to apply to the electrodes a first set of ablation pulses. Similarly, in case the first and second sections form the two disjoint ablation lines, the processor is configured to control the pulse generator to apply to the electrodes a second different set of ablation pulses.

In some embodiments, the selected electrodes of a given section are typically located at the ends of the given section they define, and are also referred to herein as end electrodes. Typically, compared to the other electrodes of the given section, the end electrodes receive less energy from the pulses applied by the pulse generator. Therefore, the processor is configured to apply a compensation mechanism, by controlling the pulse generator for supplying an even energy across the given section. For example, the processor may control the pulse generator to supply higher energy to the end electrodes. In such embodiments, in case the first and second sections jointly form a single contiguous ablation line, the processor is configured to control the pulse generator to deliver a first total energy, via the second and third electrodes. As described above, in case the processor identifies that the first and second sections form two disjoint ablation lines, the processor is configured to deliver, via the second and third electrodes, a second total energy, higher than the first total energy, so as to compensate for the larger number of end electrodes.

The disclosed techniques improve the uniformity of bipolar ablation lines in ablation procedures carried out using a catheter having multiple electrodes, and therefore, improve the uniformity of the lesion formed in the ablation procedures.

### SYSTEM DESCRIPTION

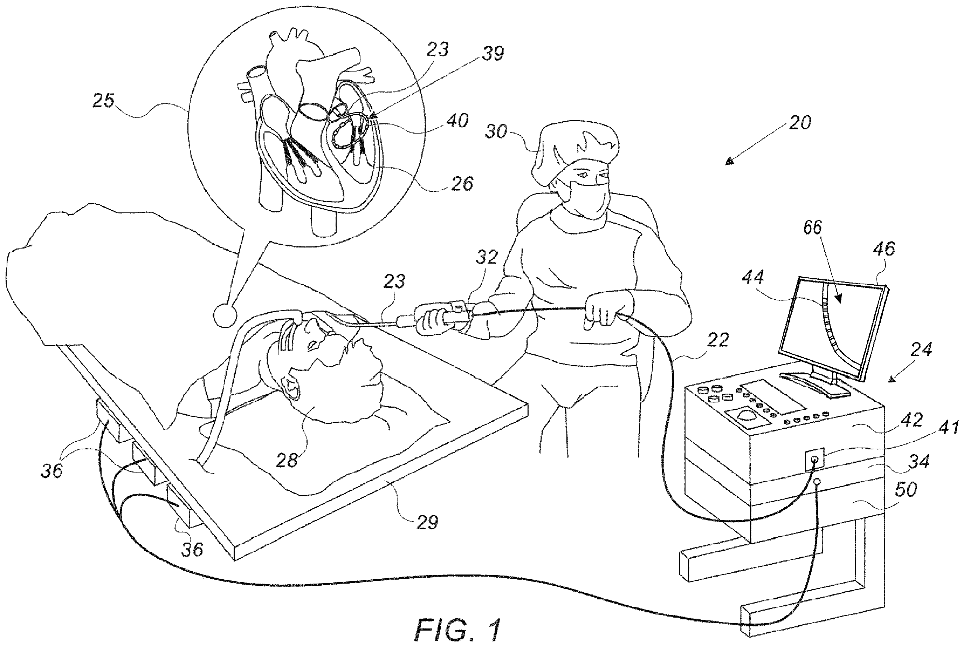

Fig. 1 is a schematic, pictorial illustration of a catheter-based position-tracking and ablation system 20, in accordance with an embodiment of the present invention. In some embodiments, system 20 comprises a catheter 22, in the present example an expandable lasso-type cardiac catheter but can also be any suitable type of a flexible catheter, and a control console 24. In the embodiment described herein, catheter 22 may be used for any suitable therapeutic and/or diagnostic purposes, such as in a bipolar radiofrequency (RF) ablation or in an irreversible electroporation (IRE), of tissue in a heart 26.

In some embodiments, console 24 comprises a processor 42, typically a general-purpose computer, with suitable front end and interface circuits for receiving signals from catheter 22 and for controlling other components of system 20 described herein. Processor 42 may be programmed in software to carry out the functions that are used by the system, and is configured to store data for the software in a memory (not shown). The software may be downloaded to console 24 in electronic form, over a network, for example, or it may be provided on non-transitory tangible media, such as optical, magnetic or electronic memory media. Alternatively, some or all of the functions of processor 42 may be carried out using an application-specific integrated circuit (ASIC) or any suitable type of programmable digital hardware components.

In some embodiments, console 24 comprises a pulse generator 50, which is controlled by processor 42 and is configured to generate one or more RF pulses to be applied, via electrodes of catheter 22, to a selected tissue of heart 26.

Reference is now made to an inset 25. In some embodiments, catheter 22 comprises a distal-end assembly 40 having a lasso-shape, and a shaft 23 for inserting distal-end assembly 40 to a target location for ablating tissue in heart 26. During an ablation procedure, physician 30 inserts catheter 22 through the vasculature system of a patient 28 lying on a table 29. Physician 30 moves distal-end assembly 40 to the target location in heart 26 using a manipulator 32 near a proximal end of catheter 22, which is connected to processor 42 via interface circuitry, referred to herein as an interface 41.

In some embodiments, catheter 22 comprises at least one position sensor 39 of a position tracking system, which is coupled to the distal end of catheter 22, e.g., in close proximity to distal-end assembly 40. In the present example, position sensor 39 comprises a magnetic position sensor, but in other embodiments, any other suitable type of position sensor (e.g., other than magnetic-based) may be used. Catheter 22 may comprise multiple position sensors 39 disposed, for example, between electrodes of distal-end assembly 40.

Reference is now made back to the general view of Fig. 1. In some embodiments, during the navigation of distal-end assembly 40 in heart 26, processor 42 receives signals from magnetic position sensor 39 in response to magnetic fields from external field generators 36, for example, for the purpose of measuring the position of distal-end assembly 40 in heart 26. In some embodiments, console 24 comprises a driver circuit 34, configured to drive magnetic field generators 36. Magnetic field generators 36 are placed at known positions external to patient 28, e.g., below table 29.

In some embodiments, processor 42 is configured to display, e.g., on a display 46 of console 24, the tracked position of distal-end assembly 40. In the present example, a segment 66 of distal-end assembly 40 displayed on an image 44.

The method of position sensing using external magnetic fields is implemented in various medical applications, for example, in the CARTO^{™} system, produced by Biosense Webster Inc. (Irvine, Calif.) and is described in detail in U.S. Patent Nos. 5,391,199, 6,690,963, 6,484,118, 6,239,724, 6,618,612 and 6,332,089, in PCT Patent Publication WO 96/05768, and in U.S. Patent Application Publication Nos. 2002/0065455 A1, 2003/0120150 A1 and 2004/0068178 A1, whose disclosures are all incorporated herein by reference.

In some embodiments, processor 42 is configured to control pulse generator 50 to apply one or more bipolar RF pulses or IRE pulses, to any electrode-pair in segment 66 of distal-end assembly 40.

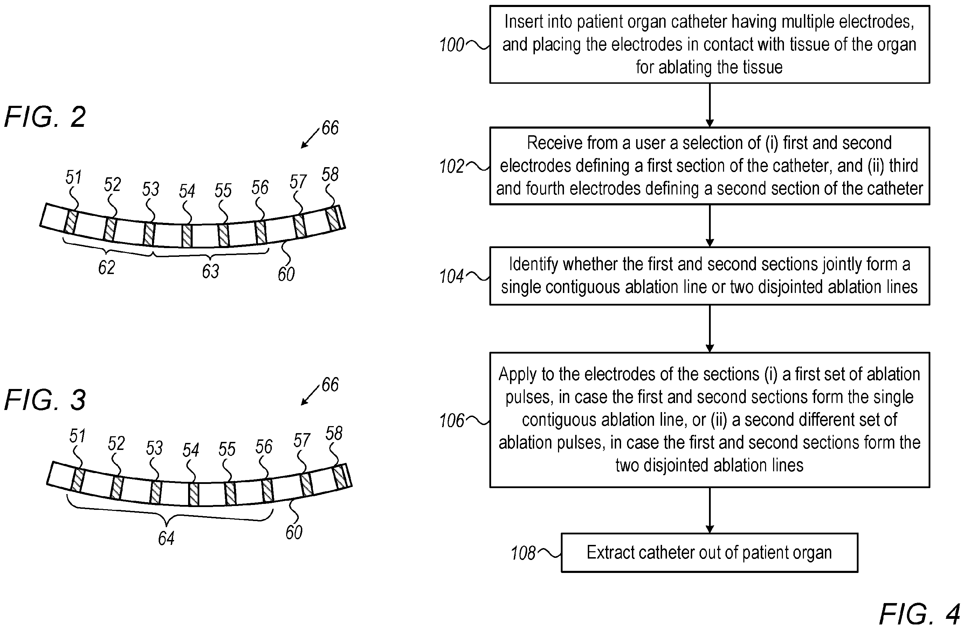

In some embodiments, interface 41 is configured to receive from a user (e.g., physician 30), a selection of multiple electrodes (typically in contact with tissue of heart 26) of distal-end assembly 40. The selected electrodes are defining one or more sections (shown in Figs. 2 and 3 below) in segment 66 for applying IRE or bipolar RF ablation pulses to the tissue of heart 26. In some embodiments, processor 42 is configured to identify whether the selected sections jointly form a single contiguous ablation line or multiple (e.g., two) disjoint ablation lines. Based on the ablation line(s) identification, processor 42 is configured to control pulse generator 50 to apply a suitable set of ablation pulses to selected electrodes of the one or more sections, so as to form a uniform lesion in the tissue of heart 26. The ablation line(s) identification and application of the one or more bipolar pulses, are described in detail in Figs. 2-4 below.

### FORMING UNIFORM ABLATION LINE IN HEART TISSUE BY ANALYZING CATHETER SECTIONS AND APPLYING SUITABLE ABLATION PULSES TO ELECTRODES OF THE SECTIONS

Fig. 2 is a schematic, pictorial illustration of sections 62 and 63 of distal-end assembly 40, in accordance with an embodiment of the present invention. In some embodiments, sections 62 and 63 comprise electrodes selected for applying bipolar ablation pulses to tissue of heart 26. As described in Fig. 1 above, the bipolar pulses may be applied for RF ablation or for IRE ablation.

In some embodiments, segment 66 of distal-end assembly 40 comprises multiple electrodes 51, 52, 53, 54, 55, 56, 57 and 58 coupled to an arm 60 of distal-end assembly 40. In the present example arm 60 is flexible, but may be rigid in other embodiments.

In some embodiments, interface 41 is configured to receive from physician 30 a selection of electrodes for performing the ablation. In the present example, the selection comprising: (i) electrodes 51 and 53, defining section 62 comprising electrodes 51-53, and (ii) electrodes 53 and 56, defining section 63 comprising electrodes 53-56.

In general, when pulse generator 50 applies bipolar ablation pulses to electrodes of a given section, electrodes at the ends of the given section, also referred to herein as end electrodes, might receive lower energy compared to that of the other electrodes of the given section. Therefore, in some embodiments, processor 42 is configured to apply a compensation mechanism, by controlling pulse generator 50 for supplying an even energy across the given section. For example, processor 42 may control pulse generator 50 to supply higher energy to the end electrodes, using various techniques described in Fig. 3 below.

In the present example, because electrode 53 is an end electrode in both selections 62 and 63, pulse generator 50 may apply the compensation mechanism twice, so that electrode 53 may receive higher than desired energy. In such cases, system 20 may produce a non-uniform ablation line causing the formation of a non-uniform lesion along segment 66, which is not desired and may be harmful to heart 26.

In some embodiments, processor 42 is configured to identify whether the sections 62 and 63 jointly form a single contiguous ablation line or two disjoint ablation lines. In case sections 62 and 63 jointly form a single contiguous ablation line, processor 42 is configured to control pulse generator 50 to apply a first set of ablation pulses to the electrodes of sections 62 and 63. Similarly, in case sections 62 and 63 form two disjoint ablation lines, processor 42 is configured to control pulse generator 50 to apply a second different set of ablation pulses to the electrodes of sections 62 and 63.

In the present example, sections 62 and 63 jointly form a single contiguous ablation line, and therefore, processor 42 is configured to control pulse generator 50 to apply the first set of ablation pulses to the electrodes of sections 62 and 63, so as to form a uniform lesion in the tissue in contact with sections 62 and 63. In some embodiments, when applying the first set of pulses to electrodes 51-56 of sections 62 and 63, electrode 53 receives the same energy applied to the other electrodes of sections 62 and 63.

In an additional example, physician 30 may select electrodes 55 and 58 for defining section 63 and may retain the selection of electrodes 51 and 53 for defining section 62. In some embodiments, based on this selection, processor 42 is configured to identify sections 62 and 63 as two disjoint ablation lines, and responsively, controls pulse generator 50 to apply a different set of pulses to the electrodes of sections 62 and 63.

In some embodiments, when applying the first set of ablation energy (e.g., when sections 62 and 63 jointly form a single contiguous ablation line as shown in Fig. 2) processor 42 is configured to control pulse generator 50 to deliver a first energy via electrode 53. When applying the second set of ablation energy (e.g., when sections 62 and 63 form two disjoint ablation lines, as described in the additional example above) processor 42 is configured to control pulse generator 50 to deliver, via electrode 53, a second total energy, higher than the first total energy (e.g., because in the disjoint ablation lines, electrode 53 serves as an end electrode receiving the energy compensation described above).

Fig. 3 is a schematic, pictorial illustration of a section 64 of distal-end assembly 40, which comprises electrodes selected for applying bipolar ablation pulses to tissue of heart 26, in accordance with another embodiment of the present invention.

In the present example, physician 30 selects electrodes 51 and 56, or two or more pairs of electrodes that mutually define section 64. For example, physician 30 may select electrodes 51 and 54 for defining a first section, and electrodes 54 and 56 for defining a second section. In some embodiments, processor 42 is configured to identify whether the first and second sections (e.g., between electrodes 51-54 and 54-56) jointly form a single contiguous ablation line (e.g., along section 64) or two disjoint ablation lines.

In some embodiments, in both selections made by physician 30 as described in Fig. 3, processor 42 is configured to identify that the selected electrodes define section 64, which forms a single contiguous ablation line. Subsequently, processor 42 is configured to control pulse generator 50 to apply bipolar ablation pulses (e.g., RF or IRE pulses) to pairs of electrodes of section 64.

In some embodiments, processor 42 is configured to control pulse generator 50 to apply, between selected pairs of electrodes of section 64, a set of bipolar ablation pulses. For example, the set of bipolar pulses may be applied, sequentially, between the following pairs of electrodes: (i) electrodes 51 and 53, (ii) electrodes 52 and 54, (iii) electrodes 53 and 55, and (iv) electrodes 54 and 56. Note that by applying this set of pulses, electrodes 53 and 54 receive the largest amount of energy, and electrodes 51 and 56 (which are end electrodes of section 64) receive the least amount of energy. Thus, assuming all pulses have the same energy, the sequence described above may form a non-uniform ablation line, and therefore, a non-uniform lesion in heart 26.

In some embodiments, processor 42 is configured to add to the sequence described above, two additional bipolar pulses applied (e.g., sequentially) between respective selected pairs of electrodes. For example, a fifth bipolar pulse between electrodes 51 and 55, and a sixth bipolar pulse between electrodes 52 and 56. Note that, compared to the lesion formed by applying bipolar pulses (i) - (iv) described above, adding the fifth and sixth bipolar pulses, improves the uniformity of the ablation line formed by electrodes 51-56 of section 64, and thereby, improves the uniformity of the lesion formed in the tissue of heart 26.

In other embodiments, processor 42 is configured to control pulse generator 50 to apply any other suitable set of ablation pulses between electrodes 51-56, so as to form a uniform ablation line along section 64. For example, bipolar pulses having a first energy (e.g., about 5 Joules) applied to electrodes 51 and 52,and between electrodes 52 and 53, and between electrodes 53 and 54, and between electrodes 54 and 55, and between electrodes 55 and 56 a second, higher energy (e.g., about 25 Joules), of bipolar pulses applied to electrodes 51 and 53, and between 52 and 54 and between electrodes 53 and 55,and between electrodes 54 and 56 and a third energy (e.g., about 35 Joules), of bipolar pulses applied to end electrodes 51 and 56.

Fig. 4 is a flow chart that schematically illustrates a method for performing a bipolar RF ablation procedure, in accordance with an embodiment of the present invention.

The method begins at a catheter insertion step 100, in which physician 30 inserts into patient organ, in the present example heart 26, catheter 22 having multiple electrodes, such as electrodes 51-58. After the insertion, physician 30 places at least some of electrodes 51-58 in contact with tissue of heart 26 for ablating the tissue.

At a user selection receiving step 102, processor 42 receives from physician 30, via interface 41, a selection of (i) electrodes 51 and 53, defining section 62 of catheter 22, and (ii) electrodes 53 and 56, defining section 63 of catheter 22. In other embodiments, physician 30 may select additional electrodes defining additional sections.

At an identification step 104, processor 42 identifies whether the sections 62 and 63 jointly form a single contiguous ablation line or two disjoint ablation lines. In case physician selects additional electrodes for defining additional sections, processor 42 identifies whether all sections jointly form a single contiguous ablation line or two or more disjoint ablation lines.

At a tissue ablation step 106, processor 42 controls pulse generator 50 for applying to the electrodes of sections 62 and 63, (i) a first set of ablation pulses, in case sections 62 and 63 form the single contiguous ablation line, or (ii) a second different set of ablation pulses, in case the sections 62 and 63 form the two disjoint ablation lines. Note that the ablation pulses are bipolar pulses applied between selected electrodes of sections 62 and 63. Moreover, the ablation may comprise RF ablation, IRE, or any other suitable type of tissue ablation.

At a catheter extraction step 108 that concludes the method, physician 30 extract catheter 30 out of heart 26 of patient 28.

Although the embodiments described herein mainly address bipolar RF-based cardiac ablation and irreversible electroporation of patient heart, the methods and systems described herein can also be used, *mutatis mutandis,* in other applications, such as in bipolar ablation of other organs, such as in renal ablation, lever ablation and lung ablation. Moreover, the embodiments described above may be used, *mutatis mutandis,* for other types of ablation, such as cryogenic ablation.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A method for improving uniformity in bipolar ablation, comprising:
inserting into a patient organ a catheter having multiple electrodes, and placing at least some of the electrodes in contact with tissue of the organ for ablating the tissue;
receiving from a user a selection of (i) first and second electrodes selected from among the electrodes, the first and second electrodes defining a first section of the catheter having two or more of the electrodes, and (ii) third and fourth electrodes selected from among the electrodes, the third and fourth electrodes defining a second section of the catheter having at least two of the electrodes;
identifying whether the first and second sections jointly form a single contiguous ablation line or two disjoint ablation lines; and
applying to the electrodes of the first and second sections (i) a first set of ablation pulses, in case the first and second sections form the single contiguous ablation line, or (ii) a second different set of ablation pulses, in case the first and second sections form the two disjoint ablation lines.

2. The method according to claim 1, wherein applying the first set of ablation pulses comprises delivering a first total energy via the second and third electrodes, and wherein applying the second set of ablation pulses comprises delivering via the second and third electrodes a second total energy, higher than the first total energy.

3. The method according to claim 1, wherein the first and second electrodes are positioned at edges of the first section, and wherein the third and fourth electrodes are positioned at edges of the second section.

4. The method according to claim 1, wherein, when the first and second sections form the single contiguous ablation line, one or more of the electrodes are common to the first and second sections.

5. The method according to claim 4, wherein the second and third electrodes comprise a same electrode.

6. The method according to claim 1, wherein, when the first and second sections form the single contiguous ablation line, the second electrode is adjacent to the third electrode.

7. The method according to claim 1, wherein the patient organ comprises a heart.

8. A system for improving uniformity in bipolar ablation, comprising:
an interface, which is configured to receive from a user a selection of (i) first and second electrodes selected from among electrodes of a catheter, wherein at least some of the electrodes are in contact with tissue of an organ for ablating the tissue, the first and second electrodes defining a first section of the catheter having two or more of the electrodes, and (ii) third and fourth electrodes selected from among the electrodes, the third and fourth electrodes defining a second section of the catheter having at least two of the electrodes; and
a processor, which is configured to: (i) identify whether the first and second sections jointly form a single contiguous ablation line or two disjoint ablation lines, and (ii) control a pulse generator to apply to the electrodes of the first and second sections (a) a first set of ablation pulses, in case the first and second sections form the single contiguous ablation line, or (b) a second different set of ablation pulses, in case the first and second sections form the two disjoint ablation lines.

9. The system according to claim 8, wherein the processor is configured to control the pulse generator to deliver a first total energy via the second and third electrodes, and wherein applying the second set of ablation pulses comprises delivering via the second and third electrodes a second total energy, higher than the first total energy.

10. The system according to claim 8, wherein the first and second electrodes are positioned at edges of the first section, and wherein the third and fourth electrodes are positioned at edges of the second section.

11. The system according to claim 8, wherein, when the first and second sections form the single contiguous ablation line, one or more of the electrodes are common to the first and second sections.

12. The system according to claim 11, wherein the second and third electrodes comprise a same electrode.

13. The system according to claim 8, wherein, when the first and second sections form the single contiguous ablation line, the second electrode is adjacent to the third electrode.

14. The system according to claim 8, wherein the patient organ comprises a heart.
